# EUROPEAN PATENT APPLICATION

(11) **EP 4 509 594 A1**
(43) Date of publication of application: **19.02.2025**
(21) Application number: 23788553.8
(22) Date of filing: 10.04.2023
(51) Int. Cl.: C12N 1/20, A61K 35/747, A61P 1/00, A23L 33/135, C12R 1/225

(54) **NOVEL MICROORGANISM AND COMPOSITION COMPRISING SAME**

(30) Priority: 12.04.2022 KR 20220045316
(71) Applicant: Seoul National University R & DB Foundation, Seoul 08826 (KR)
(72) Inventor: KO, Gwang Pyo, Seoul 06289 (KR); KIM, Woon Ki, Seoul 08826 (KR); MIN, Sung Gyu, Seoul 06624 (KR)
(74) Representative: Schön, Christoph
(86) International application number: PCT/KR2023/004816
(87) International publication number: WO 2023/200203

(57) **Abstract**

The present invention relates to a novel *Lactobacillus rhamnosus* KBL2290 strain and a composition including the same, wherein the strain exhibits various activities such as improving intestinal flora, anti-inflammatory, and strengthening intestinal tight junctions. Therefore, it is preferably used as medicines and health functional foods for intestinal-related diseases.

## Description

### [Technical Field]

The present invention relates to a novel *Lactobacillus rhamnosus* strain and a composition for improving, preventing or treating intestinal diseases, which includes the above strain.

### [Background Art]

Inside a healthy human body, approximately 100 trillion microorganisms coexist, which is about 10 times more than the number of cells in the human body. The number of genes of these microorganisms is more than 100 times that of humans, and the intestinal microflora is structured uniquely for each individual and disease. Healthy intestinal commensal microflora interacts complexly with human cells and performs a variety of functions, including metabolism, immune system development through regulation of immune responses, maintaining intestinal homeostasis and the like.

Probiotics refer to microorganisms with antibacterial and enzymatic activities that help balance of intestinal organisms, and products produced by these microorganisms. In particular, the probiotics are defined as live bacteria in the form of single or multiple strains, which are supplied to humans or animals in the form of dried cells or fermented products to improve intestinal flora. *Lactobacillus* species are known as very useful probiotics because strains capable of producing various antibacterial substances exist. For example, *Lactobacillus rhamnosus,* etc. are used, and detailed strains may include *Lactobacillus rhamnosus* GG, etc.

Inflammatory bowel disease (IBD) is a gastrointestinal disease in which chronic inflammation of unknown cause repeats improvement and recurrence, and representative examples thereof may include ulcerative colitis (UC) and Crohn's disease. Crohn's disease appears T helper type 1 (Th1)-derived immune response, for example, an increase in pro-inflammatory cytokines such as interferon-γ (IFN-γ) and tumor necrosis factor-α (TNF-α), and ulcerative colitis shows T helper type 2 (Th2)-derived immune response such as increased interleukin 13 (IL-13) as well as increased expression of Th17 cells.

Extensive research is being conducted on drug development to treat the inflammatory bowel disease, and a correlation between dysbiosis in the composition of intestinal flora and abnormal intestinal immune response has recently been revealed. As a result of analyzing the intestinal flora of patients with inflammatory bowel disease and ulcerative colitis through next-generation sequencing, it was found that, compared to healthy normal people, the patient group showed an imbalance in the intestinal flora, such as decreased diversity and dominance of *Proteobacteria.* Further, it showed characteristics in that *Ruminococcaceae* and *Lachnospiracaea,* which are associated with the production of short-chain fatty acids, are reduced, while *Enterobacteriaceae* and *Fusobacteriaceae,* which are intestinal bacteria associated with inflammation, are decreased.

Meanwhile, irritable bowel syndrome (IBS) is a condition characterized by abdominal pain and/or discomfort associated with altered bowel behavior or bowel habits, and these symptoms are not explained by structural or biochemical abnormalities. Urgency, abdominal distension, and the feeling of incomplete bowel movements are also common symptoms of IBS. Therefore, this disease is classified as a functional gastrointestinal disorder, including diseases such as functional bloating, non-cardiac chest pain, non-ulcer dyspepsia and chronic constipation or diarrhea. In particular, the IBS has a huge impact on morbidity and quality of life beyond abdominal pain and discomfort, because the associated symptoms affect in aspects of both the patient's well-being and normal functionality.

However, strains that show excellent effects in improving intestinal health including inflammatory bowel disease and irritable bowel syndrome have not yet been discovered, and relevant research is continuously being conducted.

### [Summary of Invention]

### [Problems to be Solved by Invention]

An object of the present invention is to provide live bacteria and a composition for improving intestinal health by utilizing the various activities and functionality of novel *Lactobacillus rhamnosus* strains.

### [Means for Solving Problems]

1. *Lactobacillus rhamnosus* KBL2290 strain (accession number KCTC14875BP).
2. The strain according to the above 1, wherein the strain increases intestinal flora of *Akkermansia* genus, *Lactobacillus* genus, *Ruminococcus* genus or *Prevotella* genus.
3. The strain according to the above 1, wherein the strain suppresses an expression of interleukin 1-beta (IL-1β), interleukin 2 (IL-2), interleukin 4 (IL-4), interleukin 12p40 (IL-12p40), interleukin 13 (IL-13), or interleukin 17A (IL-17A).
4. The strain according to the above 3, wherein the strain suppresses an expression of chemokine ligand 2 (CCL2) or chemokine ligand 1 (CXCL1).
5. The strain according to the above 3, wherein the strain suppresses an expression of cyclooxygenase 2 (Cox-2) or nitric oxide synthase (iNOS).
6. The strain according to the above 3, wherein the strain promotes an expression of interleukin 10 (IL-10) or transforming growth factor-β (TGF-β).
7. The strain according to the above 1, wherein the strain increases an expression of Zo-1 or Occludin.
8. A pharmaceutical composition for preventing or treating inflammatory bowel disease, including the strain according to the above 1, a culture thereof or an extract of the strain.
9. The pharmaceutical composition according to the above 8, wherein the inflammatory bowel disease includes one or more inflammatory bowel diseases selected from the group consisting of acute enteritis, ulcerative colitis, Crohn's disease, celiac disease, collagenous colitis, leaky gut syndrome and pseudomembranous colitis.
10. A pharmaceutical composition for preventing or treating functional gastrointestinal disorders, including the strain according to the above 1, a culture thereof or an extract of the strain.
11. The pharmaceutical composition according to the above 10, wherein the functional gastrointestinal disorder includes one or more selected from the group consisting of irritable bowel syndrome, functional bloating, noncardiac chest pain, non-ulcer dyspepsia, and chronic constipation and diarrhea.
12. A prebiotic composition, including the strain according to the above 1, a culture thereof or an extract of the strain.
13. The prebiotic composition according to the above 12, wherein the composition is for improving intestinal flora of at least one of *Akkermansia* genus, *Lactobacillus* genus, *Ruminococcus* genus, or *Prevotella* genus.

### [Advantageous effects]

The *Lactobacillus rhamnosus* KBL2290 strain of the present invention exhibits various activities such as improving intestinal flora, anti-inflammatory, and strengthening intestinal tight junctions. Accordingly, it is preferably used as a pharmaceutical composition for intestinal diseases, health functional food to improve intestinal flora, etc.

### [Brief Description of Drawings]

FIG. 1 illustrates analysis of *in vitro* anti-inflammatory effects of *Lactobacillus rhamnosus* KBL2290 strain, which corresponds to the results of measuring expression levels of IL-2, IFN-γ, IL-10, IL-4, IL-13, and IL-17A according to the presence or absence of treatment with the above strain.
FIG. 2 illustrates analysis of *in vivo* inflammatory bowel disease improvement effects of *Lactobacillus rhamnosus* KBL2290 strain. Specifically, FIG. 2A shows mRNA expression levels of CXCL-1, CCL-2, IL-1β, IL-12p40, COX2, iNOS, TGF-β, FOXP3 and IL-10, while FIG. 2B shows mRNA expression levels of Zo-1 and Occludin.
FIG. 3 illustrates analysis of *in vivo* inflammatory bowel disease improvement effects of *Lactobacillus rhamnosus* KBL2290 strain, which specifically corresponds to the amounts of IFNγ, IL-17A, TNF-α and IL-6 proteins according to the presence or absence of treatment with the strain of the present invention.
FIG. 4 illustrates analysis of intestinal flora improvement effects of *Lactobacillus rhamnosus* KBL2290 strain. Specifically, FIGS. 4A and 4B show analysis results based on 16S rRNA, while FIGS. 4C and 4D show distributions of intestinal flora according to the presence or absence of treatment with the strain of the present invention.
FIG. 5 illustrates analysis of short-chain fatty acid production effects of *Lactobacillus rhamnosus* KBL2290 strain, which corresponds to the production amount of acetate, butyrate and propionate.
FIG. 6 illustrates analysis of inflammatory bowel disease improvement effects of *Lactobacillus rhamnosus* KBL2290 strain. Specifically, FIG. 6A shows a change in body weight of mice due to acute enteritis, while FIG. 6B shows a change in intestine length.
FIG. 7 illustrates analysis of inflammatory bowel disease improvement effects of *Lactobacillus rhamnosus* KBL2290 strain, which specifically corresponds to the results of hematoxylin & eosin (H&E) staining of the mouse colon according to the presence or absence of treatment with the above strain.
FIG. 8 illustrates comparative analysis of *in vitro* anti-inflammatory effects of *Lactobacillus rhamnosus* KBL2290 strain with the commercial strain *Lactobacillus rhamnosus* GG (LGG) strain.

### [Mode for Carrying out Invention]

Hereinafter, the present invention will be described in detail. Unless otherwise specifically defined, all terms in the present specification would have the same meanings as general meanings of the corresponding terms understood by persons having common knowledge to which the present invention pertains, and if the general meanings conflict with the meanings of the terms used herein, the meanings used in the present specification take precedence.

The present invention relates to a novel *Lactobacillus rhamnosus* KBL2290 strain.

The strain was deposited at the Biological Resources Center, Korean Collection for Type Cultures (KCTC) on February 24, 2022, and the accession number is KCTC14875BP.

Name of depositary organization: Korean Collection for Type Cultures (KCTC)
Accession Number: KCTC 14875BP
Date of deposit: March 17, 2022

The strain may increase intestinal flora of *Akkermansia* genus, *Lactobacillus* genus, *Ruminococcus* genus or *Prevotella* genus.

The strain may reduce an expression of interleukin 1 beta (IL-1β), interleukin 2 (IL-2), interleukin 4 (IL-4), interleukin 12p40 (IL-12p40), interleukin 13 (IL-13), or interleukin 17A (IL-17A).

The strain may reduce an expression of chemokine ligand 2 (CCL2) or chemokine ligand 1 (CXCL1).

The strain may reduce an expression of cyclooxygenase 2 (Cox-2) or nitric oxide synthase (iNOS).

The strain may increase an expression of interleukin 10 (IL-10) or transforming growth factor-β (TGF-β).

The strain may increase an expression of Zo-1 or Occludin.

The strain may exhibit strong resistance to high concentrations of bile salts at low acidity such as pH 4.

The strain or a culture thereof has several excellent activities, including, for example, effects of suppressing the growth of harmful intestinal flora, effects of promoting the growth of beneficial intestinal flora, anti-inflammatory effects, effects of producing short-chain fatty acids, effects of strengthening intestinal tight junctions and the like. Accordingly, the strain, a culture thereof or an extract thereof may be used as a composition for preventing or treating inflammatory bowel disease, functional gastrointestinal disorder, leaky gut syndrome, and pseudomembranous colitis, a probiotic composition for improving intestinal function and intestinal flora. The culture may include the strain, or a culture from which the strain was isolated.

The short-chain fatty acid production effect means that the strain of the present invention or a culture thereof may produce short-chain fatty acid through decomposition of dietary fiber, and the short-chain fatty acid may include acetate, butyrate or propionate. These short-chain fatty acids may contribute to various processes associated with intestinal homeostasis, such as epithelial cell differentiation, regulatory T cell stimulation and improvement of mucosal inflammation, thereby resulting in preventive and alleviating effects on ulcerative colitis. Additionally, the above effects may be more significantly remarkable when the balance of normal bacterial flora is collapsed, such as when intestinal colitis is induced. However, it is not limited thereto.

The intestinal tight junction generally refers to the formation of a rigid defense membrane through intracellular tight junctions in the intestinal epithelial cell layer. The strain or a culture thereof of the present invention may increase the expression level of proteins involved in the tight junctions such as Zo-1 or Occludin thus to strengthen the intestinal tight junction. Alternatively, these strengthening effects may be significantly remarkable when the tight junction is weakened due to an occurrence of intestinal leakage or inflammation. However, it is not limited thereto.

Further, the present invention relates to a composition including *Lactobacillus rhamnosus* KBL2290 strain, a culture thereof or an extract thereof.

The culture of the *Lactobacillus rhamnosus* strain may include a medium (solid, liquid, etc.) containing the strain, a medium (solid, liquid, etc.) from which the strain is isolated after culturing the strain or the like.

The extract of the strain may be, for example, a lysate of the strain, and specifically may be powders obtained by lyophilizing the lysate of the strain, or a solution or dispersion thereof.

The composition may be a pharmaceutical composition for preventing or treating inflammatory bowel disease.

*Lactobacillus rhamnosus* KBL2290 strain, a culture thereof or an extract thereof has effects of inhibiting the expression of inflammatory cytokines both *in vitro* and *in vivo,* or increasing the expression of anti-inflammatory cytokines, thereby exhibiting excellent anti-inflammatory effects. The inhibitory or increasing effects may be due to transcriptional or post-transcriptional regulation of the factors at the transcription level, or may be due to translation or post-translational regulation of mRNA. However, it is not limited thereto. The inflammatory cytokines may include IL-2 as a Th1 inflammatory cytokine, IL-4 and IL-13 as Th2 inflammatory cytokines, and IL-17A as a Th17 (T helper 17) inflammatory cytokine. These inhibitory effects may suppress the immune response of Th1 and Th2 cells.

The inflammatory bowel disease corresponds to a gastrointestinal disease in which chronic inflammation repeats improvement and recurrence, and may include, for example, acute enteritis, ulcerative colitis, Crohn's disease, celiac disease, pseudomembranous colitis, collagenous enteritis, leaky gut syndrome, or pseudomembranous colitis, or the like, but it is not limited thereto. The composition may inhibit the inflammatory bowel disease by suppressing the action of Th1, Th2 or Th17, which is known to cause the inflammatory bowel disease.

Further, the composition may be a pharmaceutical composition for preventing or treating functional gastrointestinal disorders. The functional gastrointestinal disorder corresponds to a chronic disease in which chronic gastrointestinal symptoms of unknown cause persist, and may include, for example, irritable bowel syndrome, functional bloating, non-cardiac chest pain, non-ulcer dyspepsia, chronic constipation and diarrhea, etc., but it is not limited thereto. The composition may inhibit the inflammatory bowel disease by suppressing the actions of Th1, Th2 and Th17, which are known to cause the inflammatory bowel disease.

Further, the present invention relates to a probiotic composition including *Lactobacillus rhamnosus* KBL2290 strain. As described above, the strain of the present invention has beneficial effects such as inhibition of the growth of harmful intestinal flora, promotion of the growth of beneficial intestinal flora, anti-inflammatory effects, short-chain fatty acid production effects, such that the composition including the above-described strains may be used as a probiotic composition. More specifically, the composition may be used to improve the above-described inflammatory bowel disease or functional gastrointestinal disorders. However, it is not limited thereto.

Further, the present invention relates to a prebiotic composition including *Lactobacillus rhamnosus* KBL2290 strain, an extract of the strain and a culture of the strain. The extract or culture derived from the strain of the present invention may be a nutrient source helping the growth of beneficial bacteria in the intestine, thereby facilitating improvement of the intestinal environment. More specifically, while increasing the intestinal distribution of strains belonging to beneficial intestinal bacteria such as *Akkermansia* genus, *Lactobacillus* genus, *Ruminococcus* genus, and *Prevotella* genus, etc., in regard to various harmful bacteria (*Staphylococcus* genus, *streptococcus* genus, *enterococcus* genus, etc.) and other bacteria, the intestinal distribution thereof is not reduced or no significant effect is exhibited thus to improve the intestinal flora. The above effects may be more remarkable when the distribution of harmful bacteria in the intestine is relatively increased due to intestinal diseases such as acute enteritis, ulcerative colitis, Crohn's disease, celiac disease, pseudomembranous colitis, collagenous enteritis, leaky gut syndrome, or pseudomembranous colitis. For example, if the *Bacteroides* genus, which belongs to harmful bacteria, and *Mucislpirillum,* which is known to cause enteritis, are included in 40%, 50%, 60%, or 70% based on the total number of intestinal flora, the distribution of intestinal beneficial bacteria may be increased while reducing the distribution of harmful intestinal bacteria by treating with the composition of the present invention. However, it is not limited thereto.

The composition of the present invention may be administered orally, parenterally, rectally, topically, transdermally, intravenously, intramuscularly, intraperitoneally, subcutaneously or the like. Formulations for oral administration may be tablets, pills, soft and hard capsules, granules, powders, fine granules, solutions, emulsions or pellets, but they are not limited thereto. Formulations for parenteral administration may be solutions, suspensions, emulsions, gels, injections, drops, suppositories, patches, ointments, or sprays, but they are not limited thereto. The formulation may be easily prepared according to conventional methods in the field, and may further include surfactants, excipients, wetting agents, emulsification accelerators, suspending agents, salts or buffers for adjusting osmotic pressure, colorants, flavorings, stabilizers, preservatives, preserving agents or other commonly available supplements.

An application amount or administration amount of the composition of the present invention will vary depending on the age, gender, weight, pathological condition of the subject to be administered and severity of the disease, administration route, or the determination of the prescriber. Determination of the application amount based on these factors is within the level of those skilled in the art, and a daily administration dose of the active ingredient may be, for example, 0.0001 µg/kg/day to 5000 mg/kg/day, more specifically 0.00001 mg/kg/day to 15 mg/kg/day, but it is not limited thereto.

Hereinafter, the present invention will be described in detail with reference to examples.

### Example 1: Isolation of Lactobacillus rhamnosus strain

*Lactobacillus rhamnosus* KBL2290 strain was isolated from the intestinal flora of Koreans. The strain showed strong resistance to high concentrations of bile salts (2%) and at low acidity such as pH 4. The strain was cultured on *Lactobacilli* MRS Agar (BD Difco, Sparks, MD, USA) supplemented with 0.05% L-cysteine hydrochloride at 37 °C for 24 hours under anaerobic conditions. The cells were collected by centrifugation at 3000 rpm and washed twice with 1 x phosphate-buffered saline (PBS). A bacterial concentration was measured using a culture method, and bacterial colonies including 1 x PBS containing 20% glycerol were stored at -80 °C until use.

### Example 2. Analysis of mycological characteristics of Lactobacillus rhamnosus strain

### 2.1. Analysis of anti-inflammatory effects

*In vitro* experiments were conducted to confirm the immunomodulatory effect of the strain of the present invention. Human-derived PBMCs (Zen-Bio, Inc.) were cultured in RPMI-1640 medium (Gibco, Paisley UK) containing 1% penicillin/streptomycin, 1% gentamicin and 10% FBS. The cultured PBMCs (2 x 10⁵ cells) were placed in a 96-well plate, treated with 1 µg/mL (OKT3; Thermo Fisher Scientific, Inc.) of anti-CD3 antibody that activates T cells, followed by treatment with the strain of the present invention at a ratio of 1:100. Alternatively, *E. coli* was added and cultured at 37 °C for 72 hours. After strain treatment, only the supernatant of the cultured PBMC cells was collected and amounts of each cytokine were measured.

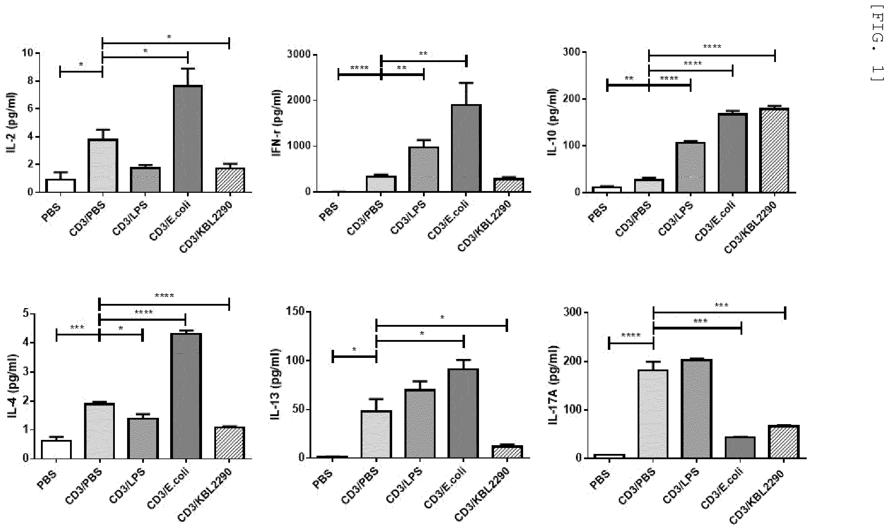

As a result, it was confirmed in FIG. 1 that the expressions of IL-2 as a Th1 inflammatory cytokine, IL-4 and IL-13 as Th2 inflammatory cytokines, and IL-17A as a Th17 inflammatory cytokine were maintained significantly lower in the KBL2290 strain treatment group (CD3/KBL2290) than those of the E. coli treatment group (*CD3*/*E.coli*) and the PBS treatment group (CD3/PBS).

### 2.1.2 Analysis of anti-inflammatory effects ( in vitro)

*In vitro* experiments were conducted to confirm the immunomodulatory effect of the strain of the present invention. Mouse bone marrow-derived dendritic cells (BMDCs) were cultured in a DMEM medium (Thermo Fisher Scientific, Inc., Waltham, MA, USA) containing 1% penicillin/streptomycin and 10% FBS. The cultured BMDCs (2.5 x 10⁵ cells) were placed in a 48-well plate, and the strain of the present invention was added thereto at a ratio of 1:20. As a control, enterohemorrhagic E. coli (*Escherichia coli* O157:H7 EC4115) isolated from the intestine was added to each well by 5 x 10⁶ cells based on the number of viable cells, and cultured at 37 °C for 24 hours. After strain treatment, the supernatant of the cultured cells was collected, and the amount of TNF cytokine was measured using BD Mouse TNF (Mono/Mono) ELISA Set II (Thermo Fisher Scientific, 558534), then RNA was extracted from the cultured cells. To this end, an easy-spin total RNA extraction kit (Intron) was used. The equal amount of extracted RNA was immediately synthesized into cDNA using a high capacity RNA-to-cDNA kit (Thermo Fisher Scientific), and then gene expression level was analyzed using a roter-gene SYBR green PCR kit (Qiagen). The primers in Table 1 below were used to target inflammatory cytokines and chemokines, anti-inflammatory cytokines, toll-like receptors, and immune checkpoint protein genes. Further, the expression level was corrected using GAPDH housekeeping gene.

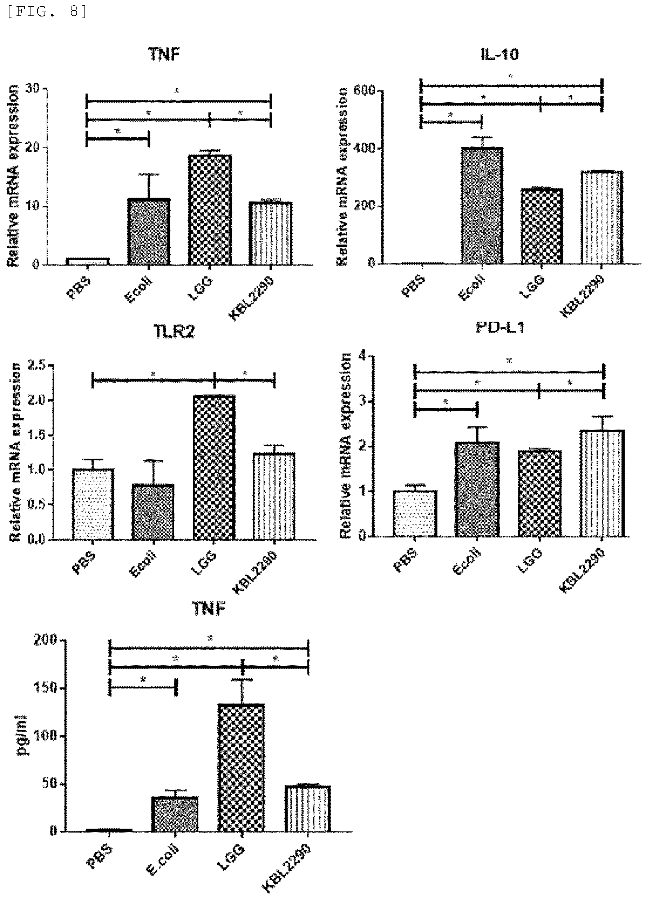

As a result, as shown in FIG. 8, it was demonstrated that the expression of inflammatory cytokine TNF and toll-like receptor TLR2 in the KBL2290 strain treatment group was significantly lower than that of the LGG treatment group at the mRNA level. Further, the expression of the anti-inflammatory cytokines, that is, IL-10, and PD-L1 as an immune checkpoint protein that lowers T-cell activity was significantly higher, as compared to LGG-treated group. Additionally, it was seen that the inflammatory cytokine TNF was significantly lower than that of the LGG-treated group at the protein level.

### 2.2. Analysis of anti-inflammatory effects and tight junction strengthening effects

In order to analyze *in vivo* anti-inflammatory effects and tight junction strengthening effects by administration of a single strain of the strain of the present invention (Seoul National University IACUC approval number: SNU-160602-9). Specifically, in order to construct a colitis mouse model, C57BL/6 7-8 week old female mice were divided into groups of 8 for each, placed in a cage having a 12-hour light/dark cycle switching setting and a constant temperature and humidity system, and prepared to be freely accessible to drinking water and food. To induce colitis, 2% dextran sulfate sodium (DSS, MP Biomedicals) was dissolved in drinking water and supplied for total 7 days. The normal control group was supplied with drinking water without added DSS, and at the same time, the control group was orally administered with 200 µL of PBS daily. Mice belonging to the experimental group were cultured with the *Lactobacillus rhamnosus* KBL2290 strain in MRS liquid medium at 37 °C under anaerobic conditions to reach the exponential phase, followed by removing the supernatant. Then, after diluting the remaining product in PBS to reach 5 x 10⁹ CFU/mL, 200 µL of diluted product was then administered daily by oral gavage from the DSS supply starting date till the end of the experiment. DSS supply was stopped after 7 days, and on the 9th day after starting DSS supply, the experiment was terminated and the mice were necropsied.

### 2.2.1. Analysis of mRNA expression level

At the end of the experiment, mouse intestinal tissue was obtained, and some colon samples were preserved in RNAlater (Thermo Fisher Scientific) solution and freeze-stored at -80 °C. In order to extract RNA, an easy-spin total RNA extraction kit (Intron) was used. The equal amount of extracted RNA was immediately synthesized into cDNA using a high capacity RNA-to-cDNA kit (Thermo Fisher Scientific), and then the gene expression level was analyzed using a roter-gene SYBR green PCR kit (Qiagen). The primers shown in Table 1 below were used to target inflammatory cytokines and chemokines, anti-inflammatory cytokines, and Zo-1 and Occludin, which are relevant to tight junctions between intestinal epithelial cells. Further, the expression level was corrected using GAPDH housekeeping gene.

**[TABLE 1]**

| Target | Sequence | SEQ ID NO. |
|---|---|---|
| ZO-1 | Fw: 5'-ACCCGAAACTGATGCTGTGGATAG-3' | 1 |
| | Rv: 5'-AAATGGCCGGGCAGAACTTGTGTA-3' | 2 |
| Occludin | Fw: 5'-ATGTCCGGCCGATGCTCTC-3' | 3 |
| | Rv: 5'-TTTGGCTGCTCTTGGGTCTGTAT-3' | 4 |
| COX2 | Fw: 5'- CACCCTGACATAGACAGTGAAAG-3' | 5 |
| | Rv: 5'- CTGGGTCACGTTGGATGAGG-3' | 6 |
| CCL2 | Fw: 5'- AGGTCCCTGTCATGCTTCTG-3' | 7 |
| | Rv: 5'- TCTGGACCCATTCCTTCTTG-3' | 8 |
| CXCL1 | Fw: 5'- TTGTGCGAAAAGAAGTGCAG -3' | 9 |
| | Rv: 5'- TACAAACACAGCCTCCCACA -3' | 10 |
| IL-12p40 | Fw: 5'- GGAAGCACGGCAGCAGAATAA -3' | 11 |
| | Rv: 5'- CTTGAGGGAGAAGTAGGAATG -3' | 12 |
| IL-1β | Fw: 5'- GAAATGCCACCTTTTGACAGTG -3' | 13 |
| | Rv: 5'- CTGGATGCTCTCATCAGGACA -3' | 14 |
| iNOS | Fw: 5'- TTTGCTTCCATGCTAATGCGAAAG - 3' | 15 |
| | Rv: 5'- GCTCTGTTGAGGTCTAAAGGCTCC G -3' | 16 |
| FOXP3 | Fw: 5'- CCCATCCCCAGGAGTCTTG -3' | 17 |
| | Rv: 5'- CCATGACTAGGGGCACTGTA -3' | 18 |
| TGFβ | Fw: 5'- GAAGGCAGAGTTCAGGGTCTT-3' | 19 |
| | Rv: 5'- GGTTCCTGTCTTTGTGGTGAA -3' | 20 |
| IL-10 | Fw: 5'- TCATTTCCGATAAGGCTTGG -3' | 21 |
| | Rv: 5'- ATAACTGCACCCACTTCCCA -3' | 22 |
| TNF | Fw: 5'- GCCACCACGCTCTTCTGCCT -3' | 23 |
| | Rv: 5'- GGCTGATGGTGTGGGTGAGG -3' | 24 |
| TLR2 | Fw: 5'- TCTAAAGTCGATCCGCGACAT -3' | 25 |
| | Rv: 5'- CTACGGGCAGTGGTGAAAACT -3' | 26 |
| PD-L1 | Fw: 5'- TGCTGCATAATCAGCTACGG -3' | 27 |
| | Rv: 5'- GCTGGTCACATTGAGAAGCA -3' | 28 |
| GAPDH | Fw: 5'- CTACAGCAACAGGGTGGTGG - 3' | 29 |
| | Rv: 5'- TATGGGGGTCTGGGATGG - 3' | 30 |

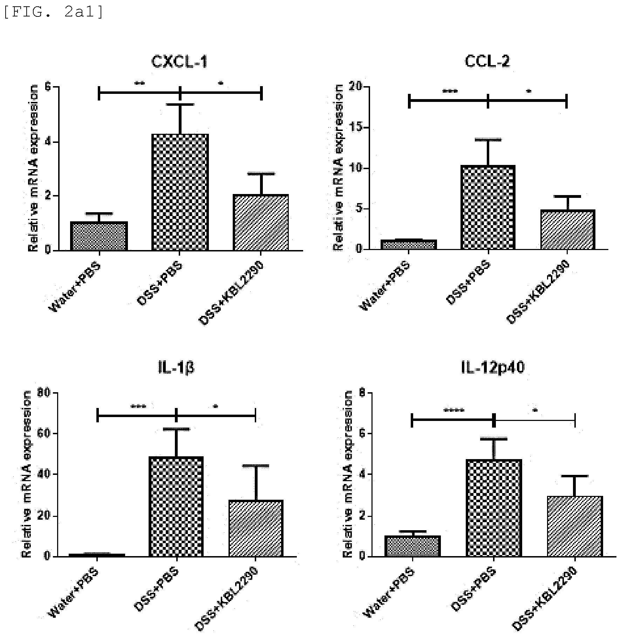

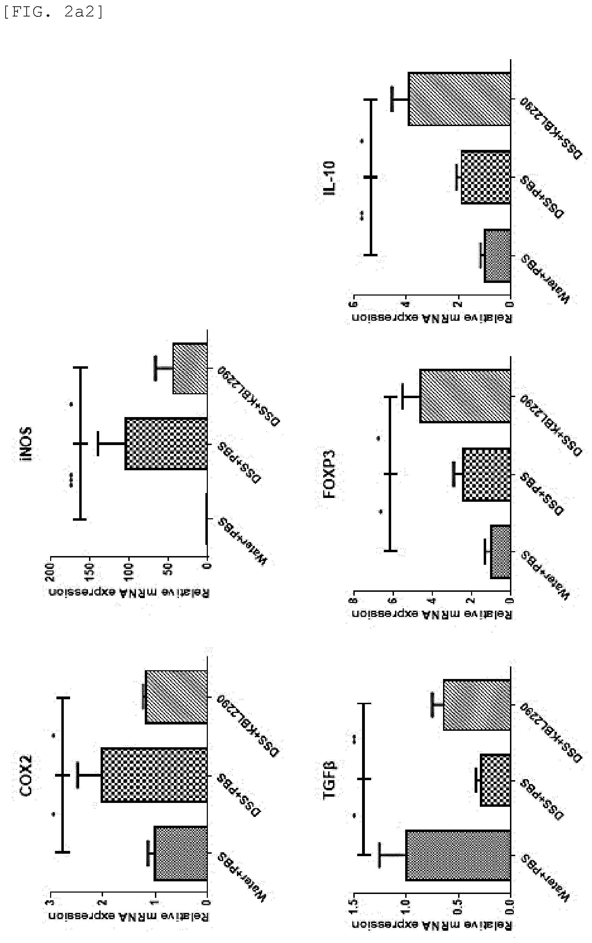

As a result, FIG 2A shows that the inflammatory cytokines IL-1β and IL-12p40, the inflammatory chemokines CCL2 and CXCL1, and the inflammatory markers COX2 and iNOS were significantly reduced in the KBL2290 administration group, compared to the DSS positive control group, in addition, the expression levels of the anti-inflammatory cytokine IL-10, TGFβ gene and FOXP3 inducing regulatory T cell differentiation were significantly increased.

This means that the *Lactobacillus rhamnosus* strain contributes to the improvement of inflammatory bowel disease *in vivo* through immune-modulation to induce IL-1β, IL-12p40, CCL2, CXCL1, IL-10 and TGFβ production, and regulatory T cell differentiation.

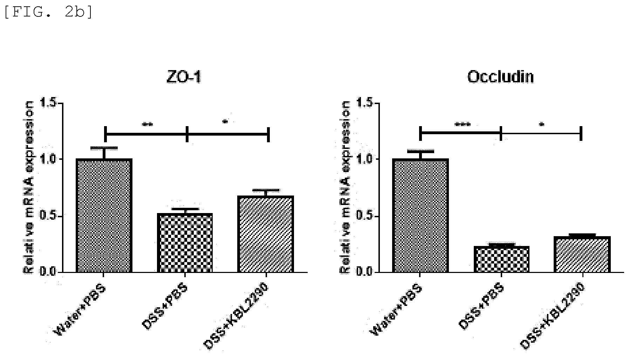

Meanwhile, in FIG. 2B, Zo-1 and *Occludin,* which are relevant to tight junctions between intestinal epithelial cells, showed a significant decrease in the expression level of both genes in the DSS positive control group compared to the normal control group. Further, as compared to the DSS positive control group, the KBL2290 administration group showed a significant decrease in the expression level of both genes. All expression levels were increased significantly. This means that administration of the strain increased the expression levels of Zo-1 and Occludin, thereby strengthening tight junctions between the intestinal epithelial cells and helping to alleviate inflammatory bowel disease *in vivo.*

### 2.2.2. Analysis of protein expression level

For tissue immunocytometry, some of the remaining colon samples were homogenized using a MM 400 Mixer Mill homogenizer (Retsch, GmbH) in 1 x RIPA buffer (Thermo Fisher Scientific) and Halt Protease Inhibitor Cocktail (Thermo Fisher Scientific), followed by centrifugation (at 4 °C and 15,000 g for 10 min) to collect the supernatant. Cytokines including IFN-γ, IL-6, IL-17A and tumor necrosis factor were measured using the BD cytometric bead array mouse Th1/Th2/Th17 cytokine kit (BD Biosciences) according to the manufacturer's instructions.

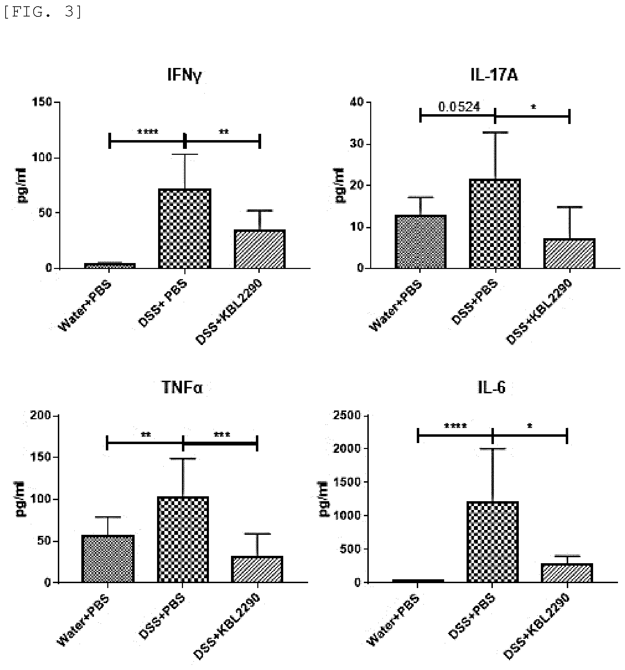

As a result, it was confirmed in FIG. 3 that major inflammatory cytokines such as IFN-γ, IL-17A, IL-6 and TNF-α were significantly reduced in the *Lactobacillus rhamnosus* KBL2290 administration group compared to the DSS positive control group.

### 2.3. Intestinal flora improvement effect

To analyze a variation in intestinal flora induced by DSS-induced colitis and colonization of a single strain, mouse feces were obtained at the end of the experiment in Example 2.2 and stored frozen at -80 °C. Total bacterial genomic DNA was extracted from the frozen sample according to the method of Example 1 above, and large-capacity sequence data was generated by targeting V4 region of the bacterial 16S rRNA gene. Using the QIIME pipeline, the entire genetic information of intestinal bacteria was confirmed to determine a structure of the mouse fecal flora, and univariate analysis (linear discriminant analysis effect size, LEfSE) was performed according to the groups.

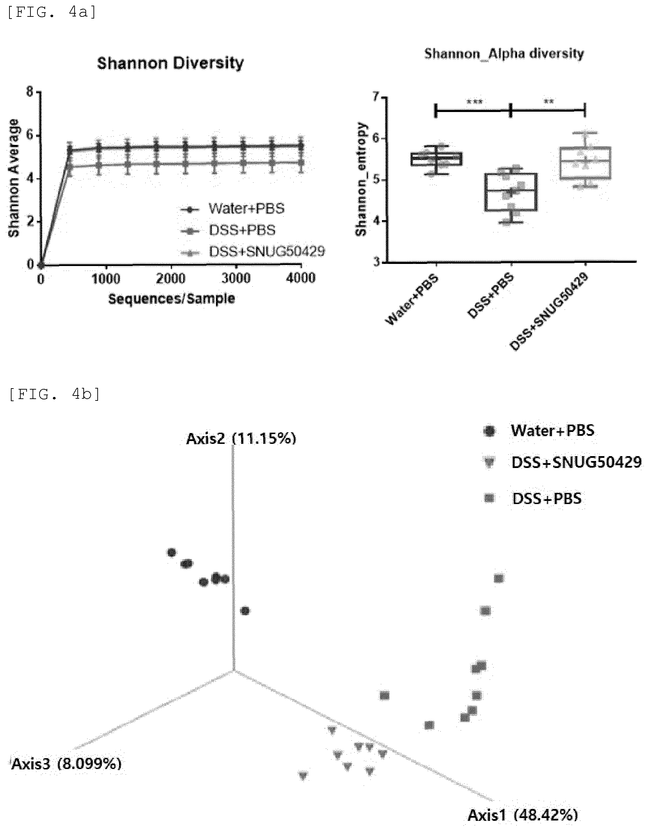

As a result, FIG. 4A shows results of determining the variation in diversity of the intestinal flora analyzed based on 16S rRNA using four alpha diversity indicators according to the groups, and it was confirmed that the diversity in intestinal flora was significantly reduced in the case of the DSS positive control group than that of the normal control group. This suggests that acute enteritis caused by DSS may have negative impact on intestinal health due to a reduction in diversity of beneficial bacteria and the dominance of potentially harmful bacteria. Meanwhile, it was confirmed that the diversity of intestinal flora in the KBL2290 administration group was significantly increased compared to the DSS positive control group. This suggests that administration of the KBL2290 strain may have positive effects on intestinal health by restoring the diversity of intestinal flora.

FIG 4B shows results of principal component analysis using weighted UniFrac distance of the intestinal flora analyzed based on 16S rRNA, and it was confirmed that the DSS positive control group had a very different intestinal flora structure from the normal control group through the PCA plot. Meanwhile, the KBL2290 administration group was confirmed to have an intestinal flora structure between the DSS positive control group and the normal control group. This suggests that administration of the KBL2290 strain changed the species making up the intestinal flora and adjusted its structure.

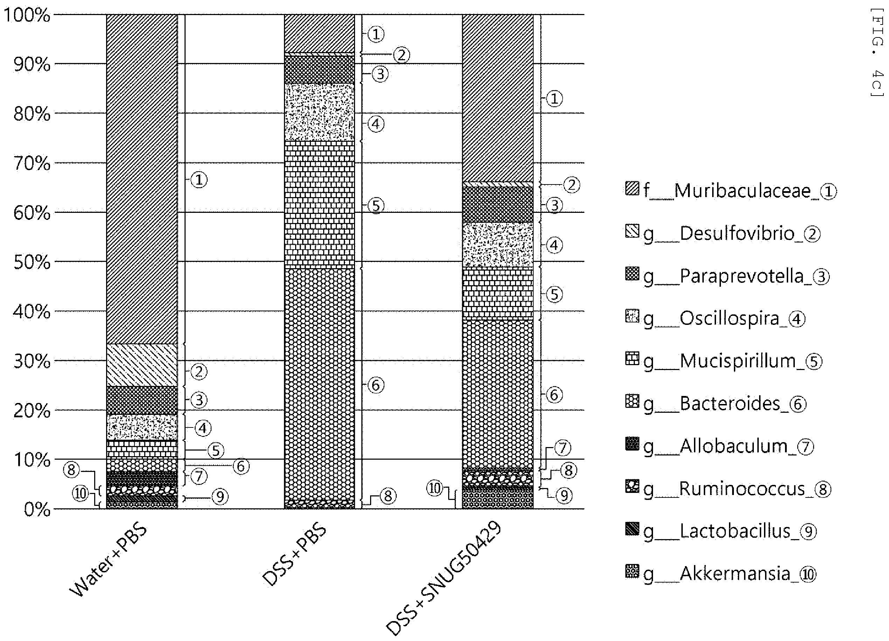

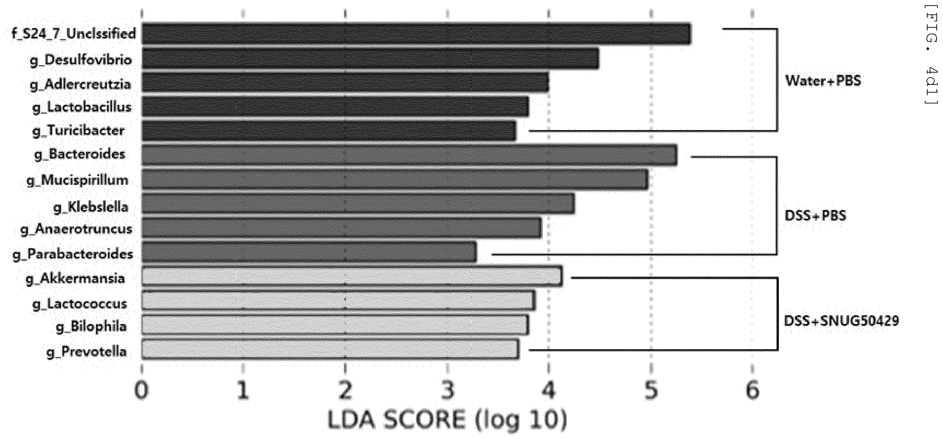

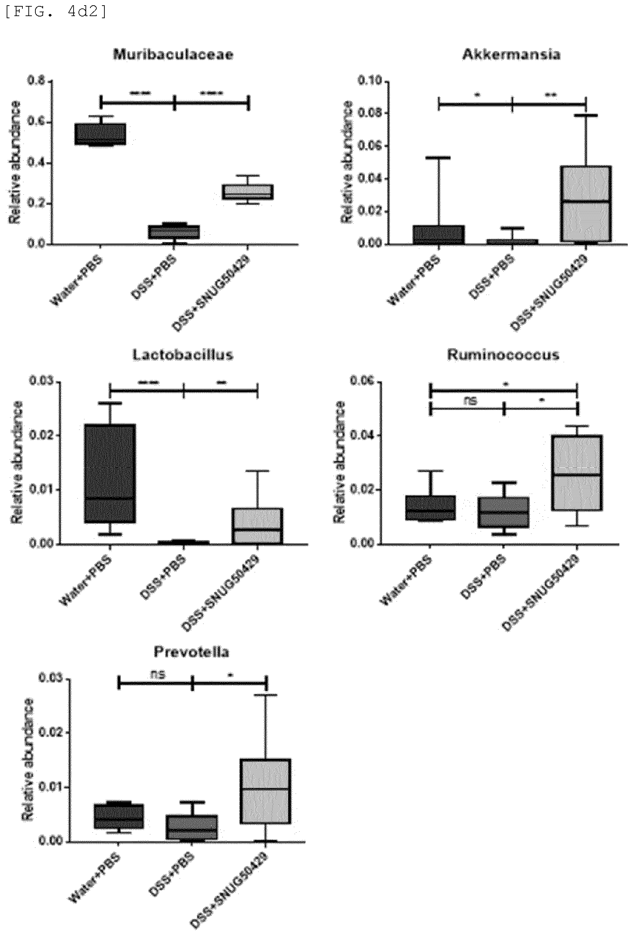

Univariate analysis (LEfSE) was performed to analyze which intestinal flora has changed in FIGS. 4C AND 4D. In the DSS positive control group, the dominance of the *Bacteroides* genus and the *Mucislpirillum* genus, which are known to cause enteritis, was confirmed. Meanwhile, in the KBL2290 administration group, increases in *Akkermansia, Lactobacillus, Ruminococcus* and *Prevotella* were confirmed. This means that administration of the KBL2290 strain helped alleviation of colitis by changing the intestinal flora.

### 2.4. Analysis of short-chain fatty acid production effects (in vivo)

To confirm the ability of the *Lactobacillus rhamnosus* KBL2290 strain to induce short-chain fatty acid production, a cecal sample was obtained at the termination of the experiment in Example 2.2, homogenized in distilled water, and centrifuged at 13,000 g for 5 minutes. The supernatant was collected, 1% 2-methylpentanoic acid was added to the supernatant as an internal standard material, and ethyl ether was used as an extraction solvent. The organic layer of the sample was measured using Agilent 7890A gas chromatograph (Agilent Technologies) under the following conditions: 1.5 kV capillary voltage; 600 L/h desolvation gas flow rate; 50 L/h cone gas flow rate; and 170 °C oven temperature; and 225 °C (flame ionization detector and injection port temperature). Nitrogen gas was used as a carrier gas. The retention time and peak area of the sample were determined using a standard mixture.

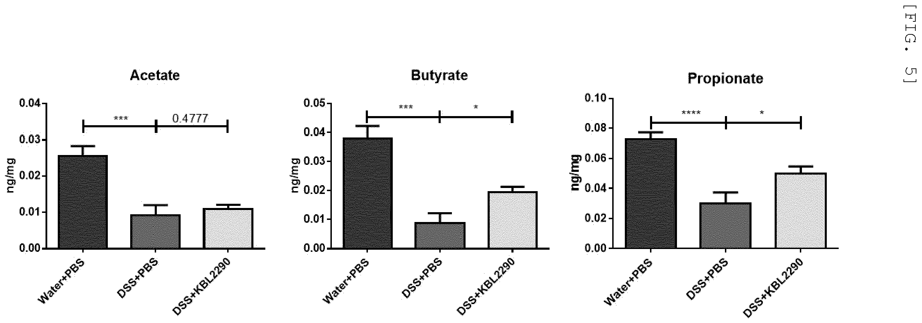

To this end, it was confirmed in FIG. 5 that short-chain fatty acid (SCFA) as a major microbial metabolite, for example, acetate, butyrate or propionate was significantly increased in the DSS+KBL2290 group administered with the strain. These short-chain fatty acids may contribute to various processes related to intestinal homeostasis, such as epithelial cell differentiation, regulatory T cell stimulation, and improvement of mucosal inflammation, thereby resulting in preventive and alleviating effects on ulcerative colitis.

### Example 3. Analysis of inflammatory bowel disease improvement effects

In order to identify whether administration of a single strain of the *Lactobacillus rhamnosus* KBL2290 strain has effects on improvement of inflammatory bowel disease *in vivo,* the body weight changes of the control group and experimental group mice were measured daily for 9 days during which enteritis caused by DSS was induced in the experiment in Example 2.2 thus to determine a change in mouse body weight due to acute enteritis. Further, after the termination of experiments, the mice were autopsied to determine a change in intestinal length.

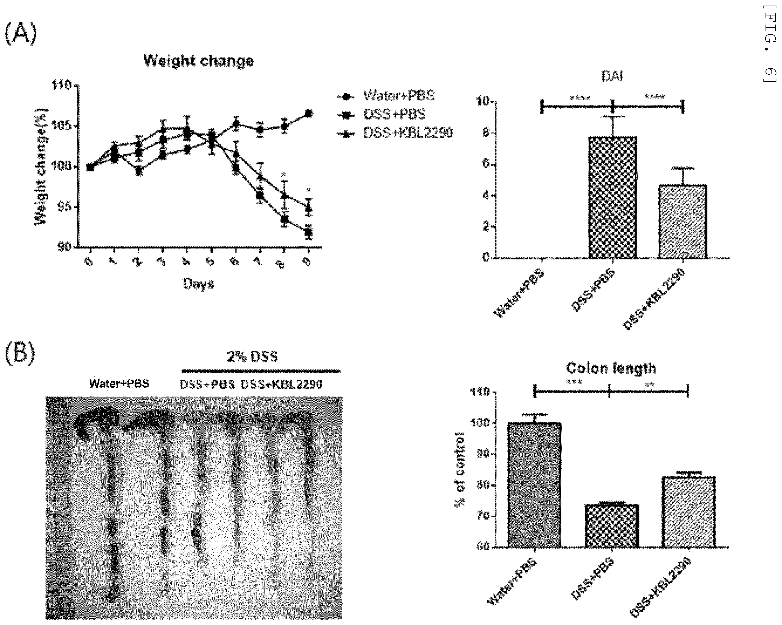

As a result, in FIG. 6A, the DSS control group showed a significant decrease in the body weight compared to the normal control group, thus confirming the construction of a colitis mouse model. Meanwhile, it was confirmed that the KBL2290 administration group showed significantly improved effects on weight loss compared to the DSS positive control group. Further, it was confirmed in FIGS. 6B that a reduction in colon length in the KBL2290 administration group was significantly improved compared to the DSS control group.

Further, hematoxylin & Eosin (H&E) staining was performed in order to observe histo-pathological variation in the colon caused by administration of a single strain of *Lactobacillus rhamnosus.* After autopsy, the distal segment of the colon was fixed in 10% neutral formalin solution, and then paraffin tissue samples were sectioned at 5 µm thickness, followed by staining with H&E reagent and observing the same under an optical microscope. The histological score of the distal segment was then examined using Panoramic Viewer (3DHISTECH, Ltd.), and the histological score was calculated and recorded in the range of 0 to 12 for four (4) categories as follows: (1) epithelial loss; (2) intestinal crypt; (3) goblet cell depletion; and (4) inflammatory cell infiltration.

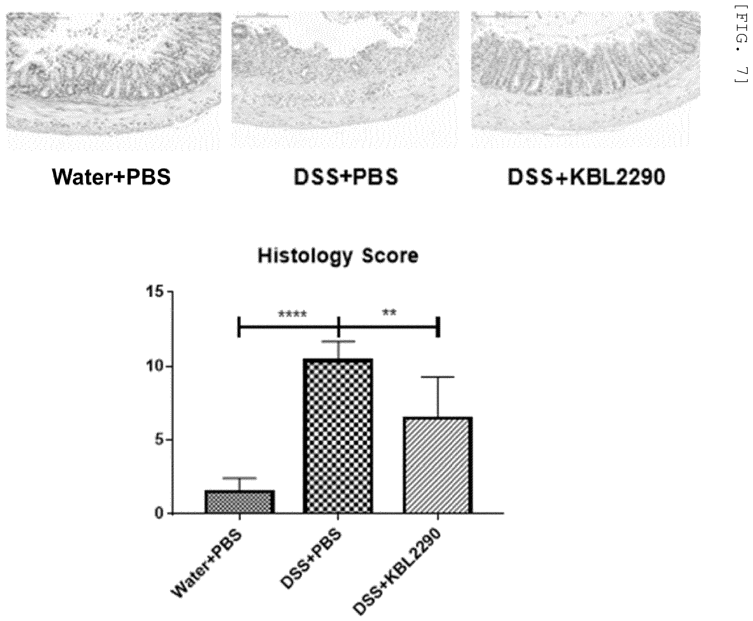

To this end, it was observed in FIG. 7 that inflammatory cells in the colon tissue were infiltrated and the mucosal layer tissue was significantly destructed in the DSS positive control group compared to the normal control group. On the other hand, histological results were confirmed such that the *Lactobacillus rhamnosus* KBL2290 administration group had alleviated inflammation compared to the DSS positive control group.

The deposit receipt of *Lactobacillus rhamnosus* KBL2290 and the translation thereof are attached below.

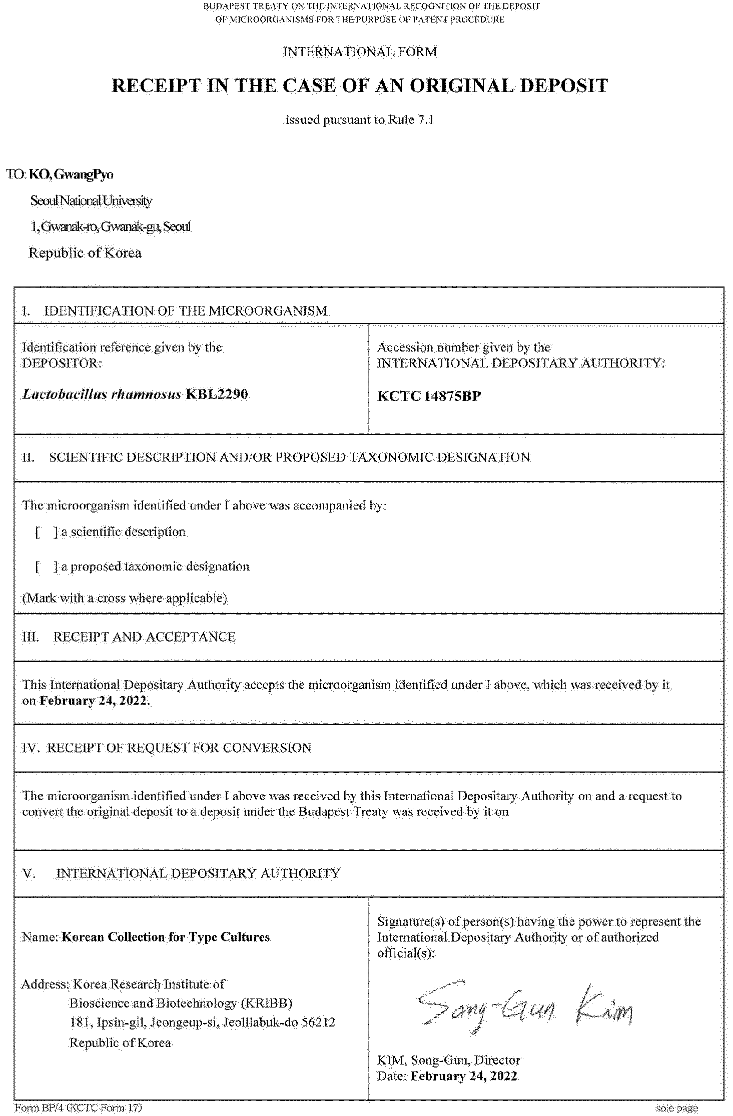
BUDAPEST TREATY ON THE INTERNATIONAL RECOGNITION OF THE DEPOSIT
OF MICROORGANISMS FOR THE PURPOSE OF PATENT PROCEDURE

INTERNATIONAL FORM

RECEIPT IN THE CASE OF AN ORIGINAL DEPOSIT

issued pursuant to Rule 7.1

TO: KO, GwangPyo
Seoul National University
1, Gwanak-ro, Gwanak-gu, Seoul
Republic of Korea

I. IDENTIFICATION OF THE MICROORGANISM
Identification reference given by the
DEPOSITOR: | Accession number given by the
INTERNATIONAL DEPOSITARY AUTHORITY:
Lactobacillus rhamnosus KBL2290 | KCTC 14875BP
II. SCIENTIFIC DESCRIPTION AND/OR PROPOSED TAXONOMIC DESIGNATION
The microorganism identified under I above was accompanied by:
[ ] a scientific description
[ ] a proposed taxonomic designation
(Mark with a cross where applicable)
III. RECEIPT AND ACCEPTANCE
This International Depositary Authority accepts the microorganism identified under I above, which was received by it
on February 24, 2022.
IV. RECEIPT OF REQUEST FOR CONVERSION
The microorganism identified under I above was received by this International Depositary Authority on and a request to
convert the original deposit to a deposit under the Budapest Treaty was received by it on
V. INTERNATIONAL DEPOSITARY AUTHORITY
Name: Korean Collection for Type Cultures

Address: Korea Research Institute of
Bioscience and Biotechnology (KRIBB)
181, Ipsin-gil, Jeongeup-si, Jeollabuk-do 56212
Republic of Korea | Signature(s) of person(s) having the power to represent the
International Depositary Authority or of authorized
official(s):
KIM, Song-Gun, Director
Date: February 24, 2022
Form BP/4 (KCTC Form 17) sole page

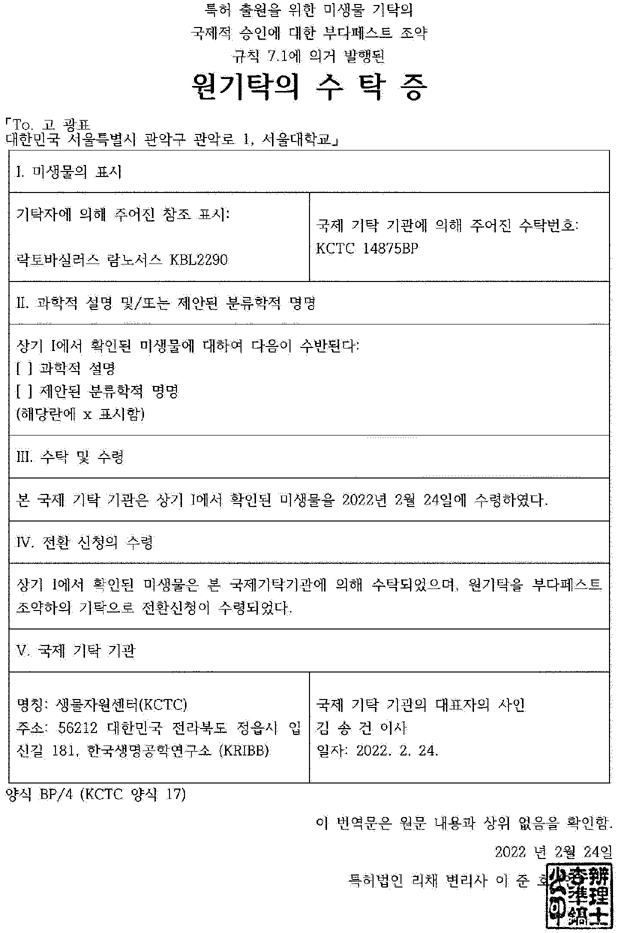
특허 출원을 위한 미생물 기탁의
국제적 승인에 대한 부다페스트 조약
규칙 7.1에 의거 발행된
원기탁의 수 탁 증

「To. 고 광표
대한민국 서울특별시 관악구 관악로 1, 서울대학교」

I. 미생물의 표시
기탁자에 의해 주어진 참조 표시:

락토바실러스 람노서스 KBL2290 | 국제 기탁 기관에 의해 주어진 수탁번호:
KCTC 14875BP
II. 과학적 설명 및/또는 제안된 분류학적 명명
상기 I에서 확인된 미생물에 대하여 다음이 수반된다:
[ ] 과학적 설명
[ ] 제안된 분류학적 명명
(해당란에 x 표시함)
III. 수탁 및 수령
본 국제 기탁 기관은 상기 I에서 확인된 미생물을 2022년 2월 24일에 수령하였다.
IV. 전환 신청의 수령
상기 I에서 확인된 미생물은 본 국제기탁기관에 의해 수탁되었으며, 원기탁을 부다페스트 조약하의 기탁으로 전환신청이 수령되었다.
V. 국제 기탁 기관
명칭: 생물자원센터(KCTC)
주소: 56212 대한민국 전라북도 정읍시 입신길 181, 한국생명공학연구소 (KRIBB) | 국제 기탁 기관의 대표자의 사인
김 송 건 이사
일자: 2022. 2. 24.
양식 BP/4 (KCTC 양식 17)

이 번역문은 원문 내용과 상위 없음을 확인함.
2022 년 2월 24일
특허법인 리채 변리사 이 준 호

## Claims

1. *Lactobacillus rhamnosus* KBL2290 strain (accession number KCTC14875BP).

2. The strain according to claim 1, wherein the strain increases intestinal flora of *Akkermansia* genus, *Lactobacillus* genus, *Ruminococcus* genus or *Prevotella* genus.

3. The strain according to claim 1, wherein the strain suppresses an expression of interleukin 1-beta (IL-1β), interleukin 2 (IL-2), interleukin 4 (IL-4), interleukin 12p40 (IL-12p40), interleukin 13 (IL-13), or interleukin 17A (IL-17A).

4. The strain according to claim 3, wherein the strain suppresses an expression of chemokine ligand 2 (CCL2) or chemokine ligand 1 (CXCL1).

5. The strain according to claim 3, wherein the strain suppresses an expression of cyclooxygenase 2 (Cox-2) or nitric oxide synthase (iNOS).

6. The strain according to claim 3, wherein the strain promotes an expression of interleukin 10 (IL-10) or transforming growth factor-β (TGF-β).

7. The strain according to claim 1, wherein the strain increases an expression of Zo-1 or Occludin.

8. A pharmaceutical composition for preventing or treating inflammatory bowel disease, comprising the strain according to claim 1, a culture thereof or an extract of the strain.

9. The pharmaceutical composition according to claim 8, wherein the inflammatory bowel disease includes one or more inflammatory bowel diseases selected from the group consisting of acute enteritis, ulcerative colitis, Crohn's disease, celiac disease, collagenous colitis, leaky gut syndrome and pseudomembranous colitis.

10. A pharmaceutical composition for preventing or treating functional gastrointestinal disorders, comprising the strain according to claim 1, a culture thereof or an extract of the strain.

11. The pharmaceutical composition according to claim 10, wherein the functional gastrointestinal disorder includes one or more selected from the group consisting of irritable bowel syndrome, functional bloating, noncardiac chest pain, non-ulcer dyspepsia, and chronic constipation and diarrhea.

12. A prebiotic composition, comprising the strain according to claim 1, a culture thereof or an extract of the strain.

13. The prebiotic composition according to claim 12, wherein the composition is for improving intestinal flora of at least one of *Akkermansia* genus, *Lactobacillus* genus, *Ruminococcus* genus, or *Prevotella* genus.
